# EUROPEAN PATENT SPECIFICATION

(11) **EP 2 496 222 B1**
(45) Date of publication and mention of the grant of the patent: **17.12.2014**
(21) Application number: 10803665.8
(22) Date of filing: 27.10.2010
(51) Int. Cl.: A61K 9/46, A61K 33/10, A61K 31/59, A61K 9/20

(54) **THE PARMACEUTICAL COMPOSITIONS COMPRISING CALCIUM AND VITAMIN D**
PHARMAZEUTISCHE ZUSAMMENSETZUNGEN MIT CALCIUM UND VITAMIN D
COMPOSITIONS PHARMACEUTIQUES COMPRENANT DU CALCIUM ET DE LA VITAMINE D

(30) Priority: 02.11.2009 TR 200908237
(43) Date of publication of application: 12.09.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000214
(87) International publication number: WO 2011/053265

(56) References cited:
- EP-A1- 1 837 019
- WO-A1-94/00107

## Description

### Field of the Invention

The present invention relates to stable and highly bioavailable pharmaceutical compositions where calcium and vitamin D are combined in a single dosage form.

### Background of the Invention

The present invention provides a composition which is effective for the prevention and treatment of the diseases related to the deficiency in calcium and vitamin D₃.

Vitamin D is a fat-soluble prohormone. Its basic forms are vitamin D₂ (ergocalciferol) and vitamin D₃ (colecalciferol) forms. Moreover, D₁, D₄, D₅ forms, the metabolites and analogs of all these substances are present. Vitamin D₃, whose chemical name is (3β, 5Z, 7E)-9,10-cekocolestra-5,7,10 (19)-trien-3-ol, is a form of vitamin D (Formula I).

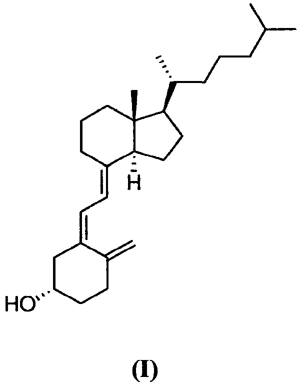

Vitamin D₃ is a naturally synthesized form of vitamin D in the body. Vitamin D3 is occured naturally in the mammal skin from 7-dehydrocholesterol under the action of UV light. It can partially be taken from animal foods. Vitamin D3 undergoes biotransformation in kidney and liver and turn into its active form of 1,25-dihydroxycholecalciferol ([1,25-(OH)₂ D₃] or calcitriol).

It plays an important role in calcium balance maintenance and parathyroid hormone regulation. It stimulates calcium renal re-absorbtion and increases the absorbtion of calcium anf phosphorus from intestinal and their mobilization from bone to plasm. It is believed that calcitriol provides the calcium absorbtion in the intestinal by binding the specific receptors that are present in the cytoplasm of mucous cells. After that, calcium is absorbed by the formation of a calcium binding protein. When the calcium and phosphorus level reach the repletion, bone mineralization occurs.

Calcium is a chemical element which is symbolized by "Ca". It is one of the minerals that are the most abundantly available in human body. 99% of calcium in the body is present in bone tissue. It plays an important role in bone structure and muscle spasm. Blood coagulation, neural transmission supply and electrical transmission in heart depend on calcium. Parathyroid hormone, vitamin D, calcitonin, glucocorticoids and magnesium can affect the calcium balance.

Calcium and its salts are used to treat or prevent the calcium deficiency. Calcium can be administered by oral route in the form of carbonate, chloride, citrate, glubionate, gluseptate, gluconate, lactate or phosphate salts; administered by parenteral route in the form of acetate, chloride, gluseptate, gluconate, glycerophosphate and lactate salts.

Minimum daily requirement of calcium in the postmenopausal women is 1500 mg. In the premenopausal women, this amount is 1000 mg. This indicates that in the in the post menopausal period a woman needs at least 500 mg / day of additional calcium supplementation.

Vitamin D is required for absorption of calcium. It was determined that vitamin D deficiency and osteoporosis that develops as a result of this is a risk factor for falls and fractures. In the postmenopausal women, due to vitamin D deficiency, calcium absorption is reduced. Various clinical studies have showed that daily calcium and vitamin D intake in men, just like in women, is lower than the recommended dose. This increases the probability of ratio of diseases (osteoporosis, osteomalacia, etc.) originating from vitamin D and calcium deficiency in adults of advanced age. Therefore, attention to dietary factors, as well as the use of various drugs supplements are also recommended

In the prior art, it is seen that the solution suggestions for providing a synergical effect are tended to the combinations comprising calcium and vitamin D. For example, the patent numbered EP 1 837 019 discloses an orally dispersible composition containing calcium and vitamin D.

However, more important thing is to combine the active substances which show effect synergistically in a single dosage form. In the patent numbered US20050261250 A1, the reason for this requirement is explained that patients do not deal with arranging the time intervals for taking two different drugs and thus they can not adjust the treatment.

According to researchs, only 50% of patients with chronic diseases in developed countries fulfill the treatment requirements. [Sabaté, E. "Adherence to Long term Therapies: Evidence for Action". World Health Organization. Geneva, 2003. 212 pp. ISBN 92-4-154599-2]. More generally, it is determined that 40-60% of people do not take their medication as prescribed and this causes bad consequences[Heneghan CJ, Glasziou P, Perera R The Cochrane Library, ISSN 1464-780X].

Patients usually skip taking their medications with the reasons of forgetfulness, busy life, different time schedules of used drugs, not finding the proper environment to drink in everytime, not understanding the illness and treatment, communication problems with the doctor, the doctor's low ability to control the patient efficiently, patients' mistrust to the doctors or the treatment, side effects, treatment perspective of the patients to illness and the treatment, depression, cognitive disorders, financial problems and a complicated treatment regimens.

A complicated dose regime, especially multiple drug use in several times a day is one of the most significant factor to prevent the patient's complience to treatment. The patient compliance and therefore the success of treatment will be higher if the treatment regimen is much simpler. For example, the combination of the active ingredients in a single dosage form will simplify treatment and increase patient's complience to treatment [Blonde L. Compliance and Persistence With Medication Therapy. Managed Care (2000). Volume 9, Number 9; archives.who.int/icium/icium2004/resources/ppt/AC016.ppt.]. The negative impact of taking multiple drug on patients will also be eliminated by the use of a combined medication in the form of a single dosage form.

However, some problems are faced during the combination of calcium and vitamin D in a single dosage form, as a result of each active agents and also combined use of these two active agents together.

The first one of these problems is bioavailability problem which caused by forming a precipitation of calcium. In the pharmaceutical formulations according to the invention, it is needed that calcium and vitamin D should be used in a wide dosage range based on the requirements. However, since the size of the tablets comprising overdose calcium will be very large, their usage will be difficult. Therefore, preferably the pharmaceutical formulations according to the invention are in the effervescent tablet form to simplify the usage. In addition, the said effervescent formulations tend to form precipitation. These precipitates formed as a result of the reaction between the calcium ions in water and citric acid when they interact, in other words the calcium citrate complexes, should be dissolved for being absorbed in body and calcium should be converted into ionized form. However, the solubility of calcium citrate complex in water is low. The most suitable environment for being dissolved of low water-soluble calcium complexes is stomach. Because, the desorption of calcium from the complexes depends on pH and calcium tends to precipitate in the environments having pH value of lower than 6.1. In this case, if the calcium complexes dissolve in stomach well, its absorbtion and thus bioavailability will be high. There are also some factors such as the remaining time of complexes in the stomach and the amount of gastric acid. It is not always possible to control these factors.

For this reason, the pharmaceutical formulations which can be produced by a repeatable manufacturing method by decreasing the formation of calcium citrate complexes to a certain extent by adding an organic acid like malic acid is more efficient way to obtain the formulations with high bioavailability instead of affecting or trying to control the said factors. There are some examples in the prior art about the pharmaceutical compositions of calcium, for example WO 94/00107 discloses an effervescent calcium supplement wherein the composition comprises calcium carbonate and edible component of citric acid and malic acid.

Moreover, it is needed that citric acid should have a lower average particle size in order to provide that citric acid interacts with less calcium ions. However, when micronized particles are converted into larger particles during the production process of the pharmaceutical dosage form, they tend to agglomerate strongly depending on the decrease in the active surface area. Micronized particles are adhered to each other intensely and wetting them is difficult. This situation results in the decrease of dissolution rate. As a result, for increasing the bioavailability, a suitable average particle size of citric acid should be determined in order to decrease the formation rate of calcium citrate complexes more and support the formation of granules which are not agglomerated.

Another problem is a turbidity problem resulting from the precipitation formed in the effervescent solutions. Considering the said interaction between the precipitation and turbidity, turbidity can be said as an indicator of the decrease in absorption and thus bioavailability. Depending on these data, in the effervescent tablets, the precipitation time after dissolution should be retarded for increasing the clearness time after dissolution and bioavailability. The solution suggestions presented by the present invention also tend to retard the precipitation time after dissolution. However, a suitable measurement method should be In of the prior art, there are generally patents which are aimed at forming complex by the composition of citric acid, malic acid and a calcium salt (calcium citrate malate). For example, in the patent numbered EP0304986 B1, calcium citrate malate in which citrate: malate mol fraction is in the range of 1:0.5 and 1:4.5; in the patent numbered EP0304987 B1, calcium citrate malate in which calcium:citrate:malate mol fraction is 6:2:3 is defined. The patent numbered EP0533724 B1 is related to the preparation method of an unstable calcium citrate malate in which its water solubility is at least 75% by weight at about 20°C. In the patent numbered EP0583378 B1 is related to a mineral supplement which comprises calcium in the range of 100-1000 mg in the form of calcium citrate malate and vitamin D form in the range of 0.6-30 µg. However, the suggestions about calcium citrate malate complex are required an unwieldy, restrictive and complicated production process. Therefore, an incomplicated production method should be determined for improving a formulation in which citric acid, malic acid and calcium are included seperately instead of forming a triple complex and for meeting the previously mentioned necessities.

The patent numbered in US5759575 A is related to the effervescent granules which comprise calcium carbonate, citric acid and another organic acid (for example malic acid) and the organic acid replace 5-20% of the part of citric acid by weight that is expected to react with calcium carbonate. However, the replaced part of citric acid for preventing it to react is in a little ratio and the precipitation can not suffiicently prevented by this little ratio. Moreover, this method is not enough alone, the particle size of the largest one of the reacting molecules should be interfered for decreasing formation of precipitation to a certain extent and increasing the solubility of the formed complexes. The change in the particle size bring about the agglomeration problem during production process. However, in the prior art, any study relative to the usage of micronized citric acid particles for both improving the solubility of calcium citrate complexes and preventing their agglomeration is not present.

However, formulations containing vitamin D are faced with stability problems because vitamin D tends to degrade upon exposure to heat, light, air, moisture, oxidizing agents or when exposed to an environment with acidic pH. This condition brings along the necessity of the selection of suitable excipients so as to ensure stability of vitamin D in tablet.

The suggestions about the stability problems of the formulations comprising calcium and vitamin D which are present prior art are generally directed to the preparation of the pharmaceutical formulations in which vitamin D is included in a protective matrix. For instance, the patent application numbered WO9906051 A1 is related to the pharmaceutical compositions comprising coated form of vitamin D in the range of 500-1000 IU, calcium in 1-2 grams and a binding agent. The patent application numbered WO2007065441 A1 is related to the pharmaceutical compositions comprising calcium compound and vitamin D, in which at least a part of the formulations is coated by film coating. However, in the said patent applications, the stability problems of vitamin D is only tried to be solved, the problems about calcium is not included. In fact, the problems arising from both calcium and vitamin D should be solved seperately for the preparation of the formulation comprising both substance.

Therefore, a new preparation methods for solving the bioavailability and stability problems of the pharmaceutical compositions comprising calcium and vitamin D which are acceptable for application and cost is still needed.

The present invention relates to the stable and highly bioavailable pharmaceutical compositions comprising calcium and vitamin D in a single dosage form which is used for prevention and treatment of metabolic bone diseases wherein the compositions increases the patient's compliance to treatment and thus treatment success.

### Summary of the Invention

The present invention is related to a pharmaceutical composition comprising calcium and vitamin D, characterized in that the composition is comprised of a biphasic mixture wherein the mixture comprises a first phase including calcium carbonate, micronized citric acid and malic acid and a second phase including vitamin D₃.

### Detailed Description of the Invention

The present invention presents stable and highly bioavailable pharmaceutical compositions which are combined in a single dosage form for increasing compliance to treatment and thus treatment success and effective for prevention and/or treatment and/ or supplementation of metabolic bone diseases.

A method which is acceptable for both application and cost is improved for obtaining calcium and vitamin D compounds with improved stability and bio-availability and pharmaceutically acceptable, non-toxic and therapeutically effective amount of the active substances and suitable amounts of the excipients are determined.

As a result of the studies, it is found that a pharmaceutical composition comprising a biphasic mixture formed by first phase including calcium carbonate, micronized citric acid and malic acid and second phase containing vitamin D₃ has both improved stability and bio-availability and the composition is effective for prevention and/or treatment and/or supplementation for metabolic bone diseases.

The pharmaceutical formulations comprising calcium and vitamin D are faced with the problems of bio-availability and stability. Bio-availability and stability problems are investigated incrementally during the preparation of the pharmaceutical formulations according to the invention.

In the first stage, the formation of calcium citrate complexes is tried to be decreased to a certain extent by adding malic acid. For this purpose, it is decided how much the part of citric acid to be displaced with malic acid to not react with calcium carbonate.

As a result of the studies, it is observed that when the added part of citric acid at least to calcium carbonate comprising first phase corresponds to an amount in the range of 40-80% by weight, adding malic acid in an amount according to the invention instead of an amount of the rest of the citric acid in the range of at least 20%, preferably 20-60% by weight decreases the formation rate of calcium citrate complexes clearly.

The said amount of citric acid in the range of at least 20% and preferably 20-60% by weight is added to the second phase. In this case, the first aspect of the invention is to decrease the formation of calcium citrate complexes by adding malic acid in an amount according to the invention instead of the amount of citric acid in the range of at least 20% and preferably 20-60% by weight.

In the second stage, it is tried that formation of calcium citrate complexes is restricted more by using citric acid having smaller particle size in the first phase wherein it interacts with calcium carbonate.

However, the usage of micronized particles brings about agglomeration problem during the production process. The micronized particles intensely adhere to each other and it is difficult to wet them. For this reason, a suitable average particle size interval of citric acid is determined in order to decrease the formation of precipitation and improve solubility properties by preventing agglomeration of calcium citrate complexes.

As a result of the studies, it is observed that the use of sieved citric acid (micronized citric acid) in the first phase wherein the first contact with calcium carbonate is occured which has an average particle size in the range of 150-350 µm and preferably 250 µm decreases the formation rate of the precipitation and provides the formation of the granules that do not agglomerate.

In this case, the second aspect of the invention is to decrease the formation of the calcium citrate complexes with the use of micronized citric acid having average particle size to be in the range of 150-350 µm and preferably 250 µm in the first phase wherein it interacts with calcium carbonate and obtain the granules that do not agglomerate.

In the third stage, it is tried to develop a production method relative to obtaining biphasic composition in which the interaction of calcium carbonate and citric acid is decreased to the minimum level. There are two mediums where calcium carbonate will interact with citric acid during the perion of the preparation of pharmaceutical composition according to the invention. The first one is the first phase comprising calcium carbonate and the second phase comprising vitamin D₃. Citric acid is present in both phase. The amount of citric acid in the range of 40-80% is present in the first phase, the remaining amount in the range of 20-60% is present in the second phase. As previously explained, calcium carbonate-citric acid interaction is prevented to optimum level by replacing the amount of citric acid according to the invention with malic acid and using micronized citric acid having average particle size in accordance with the invention. The first phase is coated by a granulation solution comprising deionized water and at least one pharmaceutically acceptable binder in order to prevent the interaction between calcium carbonate present in the first phase and citric acid present in the second phase. The first phase can optionally be re-granulated with deionized water.

As a result of the studies, it is determined that the interaction between calcium carbonate present in the first phase wherein calcium carbonate-citric acid interaction phase is reduced and citric acid in the second phase is effectively prevented resulting from coating the first phase by granulation solution.

In this case, the third aspect of the invention is to obtain a biphasic composition wherein the calcium carbonate-citric acid interaction between the first and second phase is decreased to minumum level with to help of coating the first phase by a granulation solution.

In the fourth stage, a measurement method is defined for evaluating the change in precipitation time of the effervescent tablet after dissolution.

This evaluation is related to determine how long the precipitation time can be delayed and which factors can affect the precipitation.

As explained before, the precipitation time for the formulations according to the invention is associated with average particle size of citric acid and the replacement rate of citric acid with malic acid in the first phase wherein the interaction with calcium carbonate occurs.

The provability of this association is tested by a measuring method of absorbance values of the effervescent tablets according to the invention in 150 ml water in UV-Vis Absorbation Spectrophotometer at 480 nm versus time.

As a result of the studies, it is determined that the precipitation time of the effervescent tablets in accordance with the invention comprising calcium carbonate, micronized citric acid and malic acid is delayed in 150 ml water for 30-60 min and the absorbance of these tablets are at least 0.4 in UV-Vis Absorbation Spectrophotometer at 480 nm in this time interval.

The said measuring method is present how the precipitation time after dissolution is affected by the displacement rate of citric acid with malic acid in the first phase and the average particle size in the first phase where the interaction with calcium carbonate occurs.

According to the method, adding malic acid instead of the amount of citric acid in the range of at least 20% and preferably 20-60% by weight delays the precipitation time by decreasing the formation of calcium citrate complexes.

However, the main decrease in the the formation of the calcium citrate complexes is observed by the use of micronized citric acid having average particle size in the range of 150-350 µm and preferably 250 µm.

In addition to the use of micronized citric acid having the described average particle size, adding malic acid instead of the amount of citric acid in the range of at least 20% and preferably 20-60% by weight also helps the precipitation time to be delayed.

Depending on delaying the precipitation time, bio-availibility and lucidity time clearly increase. In this case, the fourth aspect of the invention is that the precipitation time of the effervescent tablets in accordance with the invention comprising calcium carbonate, micronized citric acid and malic acid in 150 ml water is delayed for 30-60 min and the absorbance of these tablets are at least 0.4 in UV-Vis Absorbation Spectrophotometer at 480 nm in this time interval.

In the fifth stage, suitable excipient composition and application method to provide the stability of vitamin D in the tablet is determined. In the pharmaceutical compositions in accordance with the invention, the first phase comprising calcium carbonate and the secordary phase comprising vitamin D₃ are formulated separately in order to apply different solutions on the different problems faced with in each phase.

The solutions related to the calcium carbonate-citric acid interaction in the first phase and between the first phase and second phase are explained previously. The solutions for the stability problem in the second phase comprising vitamin D₃ are examined in this stage. A composition increasing stability is improved in order to control the tendency of vitamin D to degredation.

As a result of the studies, it is determined that after blending Vitamin D3 and at least one binder and/or diluent which is preferably water-soluble, not hygroscopic and stable in the solutions makes Vitamin D₃ stable by surrounding it.

Morover, at least one excipient which is resistant to degredation and has an effect of stabilizing can be added to the pharmaceutical formulation. In this case, the fifth aspect of the invention is blending vitamin D with suitable excipients to maintain the stability of vitamin D in tablet and thus surrounding of vitamin D with a protective coating.

Since the pharmaceutical formulations in accordance with the invention is preferably produced in the form of effervescent tablet, it is considered that it is also required to enhance the taste and smell of the drug. For this reason, at least one flavoring and/or aroma agent is added to both phases. Moreover, at least one disintegrant which is required for the effervescent tablets to be dispersed in water (effervescent pair comprising effervescent acid and effervescent base) is added to the first phase, which is progressively prepared with a method of solving precipitation problem, for speeding up the dissolution by starting efervescent reaction. The pharmaceutical formulations in accordance with the invention comprises citric acid and malic acid as effervescent acid in the first phase and preferably sodium carbonate and/or sodium hydrogen carbonate as effervescent base. The rest of the citric acid is added to the second phase.

In the last stage, the resulting composition is obtained by combining the first phase comprising calcium carbonate and the second phase comprising vitamin D3 and the tablets are pressed according to the specification by feeding the composition into tablet pressing machine.

It is determined that the pharmaceutical compositions in accordance with the invention in the form of effervescent tablet which is produced by applying the said production method remains stable in the accelarated stability studies (402°C ± 2°C / %75 ± %5 RH) for at least 6 months and in the long-term stability studies (252°C ± 2°C / %60 ± %5 RH) for at least 12 months.

The preparation method of pharmaceutical formulations in accordance with the invention with improved stability and bio-availability includes the following steps:
In the preparation period of the first phase;
   - Granulation solution comprising deionized water and at least one pharmaceutically acceptable binder is prepared;
   - Citric acid in an amount in the range of 40-80% by weight is sieved in a way that its average particle size will be in the range of 150-350 µm and preferably 250 µm;
   - Calcium carbonate, sieved micronized citric acid, malic acid replaced with the amount of citric acid in the range of at least 20% and preferably 20-60% by weight, at least one pharmaceutically acceptable binder and at least one flavoring and/or aroma agent is fed to the granulator;
   - The obtained composition is granulated by the granulation solution;
   - The obtained granules are dried in a way that its humidity wil be maximum 0.4%;
In the preparation period of the second phase:
   - Vitamin D3 is blended with at least one pharmaceutically acceptable binder and/or diluent;
   - The obtained blend is combined with of the rest of citric acid which is in an amount in the range of 20-60% by weight, at least one flavoring and/or aroma agent and preferably at least one expicient having stabilizing effect;
In the preparation period of the final composition:
   - The final composition is formed by adding the dried granules obtained in Step 5 after sieving and adding them in the mixture obtained in Step 7;
   - The final composition is fed into the tablet pressing machine and pressing tablets according to the specifications.

The sixth aspect of the invention is to determine the suitable composition of calcium carbonate, vitamin D3, micronized citric acid and malic acid with pharmaceutically acceptable at least one excipient which is selected from diluent, binder, disintegrant, lubricant, glidant, stabilizing agent, flavoring and aroma agent for the use of prevention and/or treatment and/or supplementation of metabolic bone diseases. The ratios given throughout the invention are the ratios by weight.

The pharmaceutical formulations in accordance with the invention is preferably formulated in the form of effervescent tablet so as to have calcium in a high level. The pharmaceutical formulations in accordance with the invention comprise calcium carbonate preferably in the range of 625-12500 mg (equivalent to 250-5000 mg elementary calcium), preferably vitamin D3 in the range of 100-100000 IU, preferably micronized citric acid in an amount up to 60% by weight and malic acid in an amount up tu 10% by weight. According to the invention, the said composition wherein calcium carbonate, vitamin D3, micronized citric acid and malic acid with pharmaceutically acceptable at least one excipient which isselected from diluent, binder, disintegrant, lubricant, glidant, stabilizing agent, flavoring and aroma agent are combined in a single dosage form has an effect on prevention and/or treatment and/or supplementation of metabolic bone diseases and increases patient's compliance to treatment and thus the treatment success.

The term of "the use for the prevention and/or treatment and/or supplementation of disease and conditions associated with metabolic bone disease" in the invention means in osteoporosis treatment; to reduce the risk of fracture in bones including vertebra and hip in the postmenopausal women; to reduce the risk of bone fractures in men in the treatment of osteoporosis; Idiopathis osteoporosis; various diseases caused by osteoporosis, and glucocorticoid and steroid mediated osteoporosis, osteopenia, osteomalacia, osteogenesis imperfecta, osteocondrodysplasia, Sudek's atrophy, rheumatoid arthritis, Paget's disease, malignant tumors of bone metastases, hypercalcaemia, or in the treatment of diseases such as hyperthyroidism; and nutritional supplements especially for woman which are in growth, pregnant or breast-feeding in the period before and after menopause to maintain bone health, but the explained usage areas are not limited with these.

The pharmaceutical formulations in accordance with the invention comprise preferably calcium carbonate salt as calcium source and the salts can be selected from chloride, citrate, glubionate, gluseptate, gluconat, lactate, phosphate, maleate, glycerophosphate, bicarbonate or tartrate.

Pharmaceutically acceptable diluents can be selected from lactose, microcrystalline cellulose, starch, pregelatinized stach, modified starch, dibasic calcium phosphate, tribasic calcium phosphate, calcium sulfate trihydrate, calcium sulfate dihydrate, calcium carbonate, kaoline, lacthylol, granulated cellulose, dextrad, dextrine, sucrose, maltose, fructose, mannitol, sorbitol and xylitol.

Pharmaceutically acceptable binders can be selected from starches such as potato starch, corn starch, wheat starch; sugars such as sucrose, glucose, dextrose, lactose, maltodextrin; natural and synthetic resins; gelatin; cellulose derivatives such as microcrystalline cellulose, HPC, HEC, HPMC, carboxymethylcellulose, methylcellulose, ethylcellulose; polyvinylpyrrolidone (povidone); polyethylene glycol (PEG); paraffin; calcium carbonate; calcium phosphate; alcohols such as sorbitol, xylitol, mannitol and water.

At least one binder and/or diluent, which are included in the pharmaceutical formulations in accordance with the invention, can be selected from sugars, PEG 6000, povidone and starches and they are preferably present in an amount up to 10% in the pharmaceutical formulation.

Pharmaceutically acceptable disintegrants can be selected from starches such as potato starch, corn starch, wheat starch, pregelatinized stach, sodium starch glucolate; cellulose derivatives such as croscarmellose sodium or microcrystalline cellulose; polyvinylpyrrolidone; crospovidone; alginic acid and its salts; crastan resin or clays like Veegum; ion-exchange resins and effervescent systems (alkaline or the alkaline earth carbonates [such as sodium carbonate, sodium hydrohen carbonate, potassium carbonate, potassium hydrogen carbonate, calcium carbonate]; water soluble polybasic organic acids or salts such as sodium hydrogen sulfate, potassium hydrohen sulfate, sodium dihydrogen phosphate, succinic acid, tartaric acid, adipic acid, citric acid).

The effervescent pair (effervescent acid and effervescent base) which is responsible for effervescent reaction is defined as "disintegrant" in pharmaceutical formulations in accordance with the invention. Pharmaceutical formulations in accordance with the invention comprise citric acid and malic acid as effervescent acid and preferably sodium carbonate and/or sodium hydrogen carbonate as effervescent base. Disintegrant is present in an amount up to 80% by weight.

Pharmaceutically acceptable lubricants can be selected from metallic stearates (such as magnesium stearate, calcium stearate, aluminium stearate), fatty acid esters (like sodium stearyl fumarate), fatty acids (like stearic acid), fatty alcohols, glyceryl behenate, mineral oil, paraffines, hydrogen vegetable oil, leucine, polyethylene glycols, metallic lauryl sulfates (such as sodium lauryl sulfate, magnesium lauryl sulfate), sodium chloride, sodium benzoate, sodium acetate and talc. The pharmaceutical compositions according to the invention preferably includes sodium stearyl fumarate as a lubricant. The lubricant is preferably present in the composition in an amount up to 5% by weight.

Pharmaceutically acceptable glidants can be selected from silicon dioxide, magnesium trisilicate, granuled cellulose, starch, talc, tribasic calcium phosphate, metallic sterates, calcium silicate and metallic lauryl sulfates. The gildant is preferably present in an amount up to 1% by weight in the pharmaceutical formulations.

Pharmaceutically acceptable stabilizing agent and/or agents can be selected from antioxidants, chelating agent, alkalizing agents and and photo-protectors. The stabilizing agent and/or agents are preferably present in an amount up to 0-85% by weight, more preferably in an amount up to 0.1-75% by weight in the pharmaceutical formulations.

Antioxidants can be selected from some substances such as butylated hydroxyanisole (BHA), sodium ascorbate, butylated hydroxytoluene (BHT), sodium sulfite, gallates (like propil gallates), tocoferole, citric acid, malic acid, ascorbic acid, acetylcysteine, fumaric acid, lecithin, ascorbil palmitate, ethylenediamine tetraacetate.

Chelating agents can be selected from disodium EDTA, edetic acid, citric acid, sodium citrate, potassium citrate or their combinations.

Alkalizing agents can be selected from alkali metal salts such as sodium carbonate, sodium hydrogen carbonate, sodium hydroxide, sodium silicate, disodium hydrogen orthophosphate, sodium aluminate; the alkaline earth metal salts such as calcium carbonate, calcium hydroxide, dibasic calcium phosphate, calcium sulfate, calcium acetate, calcium gluconate, calcium glycerophosphate, magnesium carbonate, magnesium hydroxide, magnesium sulfate, magnesium acetate, magnesium silicate, magnesium aluminate and first, second and tertiary amines, cyclic amines, N,N'-dibenzylethylenediamin, dietanolamine, ethylenediamin, meglumine, monosodium glutamate, polacrilin sodium, sodium alginate.

Photo-protecting agents can be selected from metal oxides such as titanium oxide, iron oxide or zinc oxide.

Pharmaceutically acceptable flavors can be selected from sucralose, sucrose, fructose, glucose, galactose, cheilosis, dextrose, levulose, lactose, maltose, maltodextrin, mannitol, maltitol, maltol, sorbitol, xylitole, erythritol, lactitol, isomalt, corn syrup, saccharine, saccharine salts, asesulpham potassium, aspartam, D-tryptophane, monoamonium glycirizinate, neohesperidin dihydrocalcon, taumatin, neotam, alitam, steviocit and cyclamate. Flavors are preferably present in an amount up to 5% by weight in the pharmaceutical formulations.

Pharmaceutically acceptable aroma agents can be selected from natural aroma oil (such as peppermint oil, oil of wintergreen, carnation bud oil, parsley oil, eucalyptus oil, lemon oil, orange oil), menthol, menthane, anethol, salicylate, eucalyptole, cinnamon, 1-methyl acetate, sage, eugenol, oxanon, alpha-irison, marjoram, lemon, orange, propenyl guaetol acetyl, cinnamon, vanilla, thymol, linalol, cinnamaldehyde glycerol acetate, N-stated p-mentan-3-carbosamide, 3,1- methoxy propane 1,2-diol. Aroma agents are preferably present in an amount up to 5% by weight in the pharmaceutical formulations.

In addition to them, other pharmaceutically acceptable excipients such as surface active agent, solubility modulators, electrolytes and coating agents can also be used in the formulations.

The pharmaceutical forms according to the invention can be dosage forms such as tablet, capsule, fast-disintegrating tablet, effervescent granule, fast-disintegrating granule, fast-disintegrating powder mixture and dried powder mixture for the preparation of syrup and are preferably effervescent tablet form.

The examples of pharmaceutical formulation and preparation method in accordance with the invention are represented below. These examples are given only to explain the invention and invention is not limited to these examples.

### EXAMPLES

### Example 1. The pharmaceutical composition comprising 1000 mg calcium and 880 IU Vitamin D₃.

| **Content** | **Amount (mg)** |
|---|---|
| **Calcium Carbonate** * | 2500.00 |
| **Vitamin D **** | 8.80 |
| **(over + %10)** | 0.88 |
| *Citric Acid Anhydr* | *3579.30* |
| *Malic acid* | *560.00* |
| Sodium hydrogen carbonate | 300.00 |
| Lactose monohydrate | 302.12 |
| Povidone | 108.20 |
| PEG 6000 | 155.70 |
| Sodium saccharine | 25.00 |
| Sodium cyclamate | 50.00 |
| Orange aroma | 10.00 |
| **Total tablet weight** | 7600 |

| | |
|---|---|
| * equivalent to 1000 mg Calcium ** equivalent to 880 IU Vitamin D₃ | |

Granule comprises about 100,000 IU per 1 gram.

### Example 2. The pharmaceutical composition comprising 600 mg calcium and 400 IU Vitamin D₃.

| **Content** | **Amount (mg)** |
|---|---|
| **Calcium Carbonate** * | 1500.00 |
| **Vitamin D **** | 4.00 |
| **(over + % 15)** | 0.60 |
| *Citric Acid Anhidr* | *2640.40* |
| *Malic acid* | *185.00* |
| Sodium hydrogen carbonate | 95.00 |
| Sodyum carbonate anhydr | 45.00 |
| Maltodextrin | 44.80 |
| Glycerin | 0.20 |
| Sodium saccharine | 25.00 |
| Sodium cyclamate | 50.00 |
| Orange aroma | 10.00 |
| **Total tablet weight** | 4600 |

| | |
|---|---|
| * equivalent to 600 mg Calcium ** equivalent to 400 IU Vitamin D₃ | |

### Example 3. The pharmaceutical composition comprising 1200 mg calcium and 800 IU Vitamin D₃.

| **Content** | **Amount (mg)** |
|---|---|
| **Calcium Carbonate *** | 3000.00 |
| **Vitamin D **** | 8.00 |
| **(over + %10)** | 0.80 |
| *Citric Acid Anhydr* | 3880.60 |
| *Malic acid* | 650.00 |
| Sodium hydrogen carbonate | 420.00 |
| Sucrose | 378.20 |
| Corn starch | 2.40 |
| Gelatin | 175.00 |
| Sodium saccharine | 25.00 |
| Sodium cyclamate | 50.00 |
| Orange aroma | 10.00 |
| **Total tablet weight** | 8600 |

| | |
|---|---|
| * equivalent to 1200 mg Calcium ** equivalent to 800 IU Vitamin D₃ | |

### Example 4. The production method of pharmaceutical formulation comprising 1000 mg calcium and 880 IU Vitamin D₃

The preparation period of the first phase;
1. The granulation solution comprising 500 ml deionized water and 86.40 mg povidone is prepared;
2. 1485 mg citric acid anhydrous is sieved in a way that its average particle size will be 250 µm
3. 2500 mg calcium carbonate, sieved micronized citric acid, 560 mg malic acid, 300 mg sodium hydrogen carbonate, 50 mg sodium cyclamate and 25 mg sodium saccharine are fed into the granulator;
4. The obtained composition is granulated by the granulation solution;
5. The obtained granules are dried in a way that its humidity wil be maximum 0.4%;

In the preparation period of the second phase;
6. Vitamin D3 with 155.70 mg PEG 60000, 21.80 mg povidone, 302.12 mg lactose monohydrate are blended;
7. The obtained blend is combined with 2094.30 mg citric acid and 10 mg orange aroma;

In the preparation period of the final composition;
8. The final composition is formed by adding and mixing the dried granules obtained in the first phase after sieving to the composition obtained in the second phase;
9. The final composition is fed into the tablet pressing machine and tablets are pressed according to the specifications.

## Claims

1. A pharmaceutical composition comprising calcium and vitamin D₃, **characterized in that** the composition comprises a biphasic combination of
- a first phase including calcium carbonate, citric acid and malic acid wherein the citric acid is micronized and,
- a second phase containing vitamin D₃ and citric acid in an amount of at least 20% by weight.

2. The pharmaceutical composition according to Claim 1, wherein the composition comprises micronized citric acid in the first phase having average particle size in the range of 150-350 µm, preferably 250 µm.

3. The pharmaceutical composition according to Claim 1, wherein the first phase of the composition comprises malic acid which is used instead of the amount of citric acid in the range of at least 20% and preferably 20-60% by weight.

4. The pharmaceutical composition according to Claim 1, wherein the first phase of the composition comprises micronized citric acid in the range of 40-80% by weight.

5. The pharmaceutical composition according to Claim 1, wherein the second phase of the composition comprises citric acid preferably in the range of 20-60% by weight.

6. The pharmaceutical composition according to Claim 1, comprising at least one pharmaceutically acceptable excipient selected from diluent, binder, disintegrant, lubricant, glidant, stabilizing agent, flavoring and aroma agent.

7. The pharmaceutical composition according to Claim 6, wherein the stabilizing agent is selected from antioxidants, chelating agents, alkalizing agents and photo-protectors.

8. The pharmaceutical composition according to Claim 1, wherein said composition comprises
- calcium carbonate in the range of 625-12500 mg (equivalent to elemental calcium in the range of 250-5000 mg)
- vitamin D₃ in the range of 100-100000 IU,
- micronized citric acid in an amount up to 60% by weight,
- malic acid in an amount up to 10% by weight,
- pharmaceutically acceptable at least one excipient chosen from diluent, binder, disintegrant, lubricant, glidant, stabilizing agent, flavoring and aroma agent.

9. The pharmaceutical composition according to Claim 1, wherein at least one disintegrant is citric acid and malic acid as effervescent acid and preferably sodium carbonate and/or sodium hydrogen carbonate as effervescent base.

10. The pharmaceutical composition according to Claim 9, wherein the disintegrant is preferably present in an amount up to 80% by weight.

11. The pharmaceutical composition according to claim 1, wherein said composition is in the dosage forms such as tablet, capsule, fast-disintegrating tablet, effervescent granule, fast-disintegrating granule, fast-disintegrating powder mixture and dried powder mixture for the preparation of syrup.

12. The pharmaceutical composition according to Claim 11, wherein said composition is in effervescent tablet form.

13. The pharmaceutical composition according to Claim 12, wherein the precipitation time in 150 ml water is delayed and the absorbance of these tablets are at least 0.4 in UV-Vis Absorbation Spectrophotometer at 480 nm in this time interval.

14. The pharmaceutical composition according to Claim 1, for use in the prevention and/or treatment and/or supplementation of metabolic bone diseases.

15. A method for the preparation of the pharmaceutical composition according to any one of the preceding claims, wherein
In the preparation period of the first phase:
1) Granulation solution comprising deionized water and at least one pharmaceutically acceptable binder is prepared;
2) Citric acid in an amount in the range of 40-80% by weight is sieved in a way that its average particle size will be in the range of 150-350 µm and preferably 250 µm;
3) Calcium carbonate, sieved micronized citric acid, malic acid replaced with the amount of citric acid in the range of at least 20% and preferably 20-60% by weight, at least one pharmaceutically acceptable binder and at least one flavoring and/or aroma agent is fed to the granulator;
4) The obtained composition is granulated by the granulation solution;
5) The obtained granules are dried in a way that its humidity wil be maximum 0.4% ;
In the preparation period of the second phase:
6) Vitamin D₃ is blended with at least one pharmaceutically acceptable binder and/or diluent;
7) The obtained blend is combined with of the rest of citric acid which is in an amount in the range of 20-60% by weight, at least one flavoring and/or aroma agent and preferably at least one expicient having stabilizing effect;
In the preparation period of the final composition:
8) The final composition is formed by adding the dried granules obtained in Step 5 after sieving and adding them in the mixture obtained in Step 7;
9) The final composition is fed into the tablet pressing machine and tablets are pressed according to the specifications.

## Patentansprüche

1. EinepharmazeutischeZusammensetzung, die Kalzium und Vitamin D3 beinhaltet und dadurchgekennzeichnet, dass die ZusammensetzungeinebiphasischeKombination von
- einerersten Phase, einschließlichCalciumcarbonat,Zitronensäure und Apfelsäure, wobei die Zitronensäuremikronisiertwird und,
- einerzweiten Phase, die Vitamin D3 und Zitronensäure in einerMenge auf mindestens 20 Gewichtsprozentbeinhaltet, umfasst.

2. Pharmazeutische Zusammensetzung gemäß Anspruch 1, wobei in der ersten Phase die Zusammensetzung mikronisierte Zitronensäure mit einer durchschnittlichen Teilchengröße im Bereich von 150-350 µm, vorzugsweise 250 µm beinhaltet.

3. Pharmazeutische Zusammensetzung gemäß Anspruch 1, wobei die erste Phase der Zusammensetzung die Äpfelsäure beinhaltet, die statt der Menge der Zitronensäure im Bereich von mindestens 20 und vorzugsweise 20-60 Gewichtsprozent verwendet wird.

4. Pharmazeutische Zusammensetzung gemäß Anspruch 1, wobei die erste Phase der Zusammensetzung mikronisierte Zitronensäure im Bereich von 40-80 Gewichtsprozent beinhaltet.

5. Pharmazeutische Zusammensetzung gemäß Anspruch 1, wobei die zweite Phase der Zusammensetzung Zitronensäure vorzugsweise im Bereich von 20-60 Gewichtsprozent beinhaltet.

6. PharmazeutischeZusammensetzung gemäß Anspruch 1, die mindestens einen pharmazeutisch annehmbaren Hilfsstoff beinhaltet, der aus Verdünnungsmittel, Bindemittel, Sprengmittel, Schmiermittel, Gleitmittel, Stabilisierungsmittel, Geschmacksstoffe und Aromamittel ausgewählt wird.

7. PharmazeutischeZusammensetzung gemäß Anspruch 6, wobei das Stabilisierungsmittel aus Antioxidantien, Chelatbildner, Alkalisierungsmittel und Foto- Protektoren ausgewählt wird.

8. PharmazeutischeZusammensetzung gemäß Anspruch 1, wobei die genannte Zusammensetzung umfaßt:
- KalziumkarbonatimBereich von 625 bis 12.500 mg (äquivalentzuelementaremKalziumimBereich von 250-5000 mg)
- Vitamin D3 imBereich von 100-100000 IU,
- mikronisierte Zitronensäure in einerMengebiszum 60 Gewichtsprozent,
- Äpfelsäure in einerMengebiszum 10 Gewichtsprozent,
- PharmazeutischannehmbarenmindestenseinenHilfsstoff, der aus Verdünnungsmittel, Bindemittel, Sprengmittel, Schmiermittel, Gleitmittel, Stabilisierungsmittel, Geschmacksstoffe und Aromamittelausgewähltwird.

9. PharmazeutischeZusammensetzunggemäßAnspruch 1, wobeimindestenseinSprengmittelZitronensäure und ÄpfelsäurealsBrausesäure und vorzugsweiseNatriumcarbonat und / oderNatriumhydrogencarbonatalsBrausegrundlageist.

10. PharmazeutischeZusammensetzunggemäßAnspruch 9, wobei das Sprengmittelvorzugsweise in einerMengebiszum 80 Gewichtsprozentvorhandenist.

11. PharmazeutischeZusammensetzunggemäßAnspruch 1, wobei die genannte Zusammensetzung in DosierungsformenwieTabletten, Kapseln, schnellzerfallendeTablette, Brausegranulat, schnellzerfallendesGranulat, schnellzerfallendePulvermischung und getrocknetePulvermischungzurHerstellung von Sirup ist.

12. PharmazeutischeZusammensetzunggemäßAnspruch 11, wobei die genanntezusammensetzung in Brausetablettenformist.

13. PharmazeutischeZusammensetzunggemäßAnspruch 12, wobei die Fällungszeit in 150 ml Wasserverzögertwird und die AbsorbanzdieserTablettenmindestens 0,4 in UV-Vis Absorption Spektralphotometerbei 480 nm in diesemZeitintervallist.

14. PharmazeutischeZusammensetzunggemäßAnspruch 1, zurVerwendungbei der Prävention und /oderBehandlung und / oderErgänzungmetabolischerKnochenerkrankungen.

15. EinVerfahrenzurHerstellung der pharmazeutischenZusammensetzunggemäßeinem der vorhergehendenAnsprüche, wobei
In der Vorbereitungszeit der ersten Phase:
1) GranulationslösungumfassendentionisiertesWasser und mindestenseinpharmazeutischakzeptablesBindemittelwirdhergestellt;
2) Zitronensäure in einerMengeimBereich von 40-80 Gewichtsprozentwird so gesiebt, dassihredurchschnittlicheTeilchengrößeimBereich von 150-350 µm und vorzugsweise 250 µm seinwird.
3) Kalziumkarbonat, gesiebtemikronisierte Zitronensäure, Äpfelsäure, die mit der Menge der ZitronensäureimBereich von mindestens 20 und vorzugsweise 20-60Gewichtsprozentersetztwird, mindestenseinpharmazeutischannehmbaresBindemittel und mindestenseinGeschmacks- und / oderAromamitteldem Granulator zugeführtwird;
4) Die erhalteneZusammensetzungwirddurch die Granulationslösunggranuliert;
5) Die erhaltenenGranulawerden in einer Weise getrocknet, dassihreFeuchtigkeit maximal %0,4 seinwird;
In der Vorbereitungszeit der zweiten Phase:
6) Vitamin D3 wirdmitmindestenseinempharmazeutischannehmbarenBindemittel und / oderVerdünnungsmittelvermischt;
7) Die erhalteneMischungwirdmit der restlichenZitronensäurein der Mengeim Bereich von 20-60 Gewichtsprozent, mindestenseinemGeschmacks- und / oderAromamittelund vorzugsweisemindestenseinemHilfsstoffmitstabilisierenderWirkungkombiniert;
In der Vorbereitungszeit der endgültigenZusammensetzung:
8) Die endgültigeZusammensetzungwirddurchZugabe der getrocknetenGranulagebildet, die in Schritt 5erhaltenwerden und durchZugabezu der Mischung, die in Schritt 7 erhaltenwird.
9) Die endgültigeZusammensetzungwird in die Tablettenpressmaschinezugeführt und die Tabletten wurden nach den Spezifikationengedrückt.

## Revendications

1. Une composition pharmaceutique comprenant du calcium et de la vitamine D₃, **caractérisée en ce que** la composition comprend une combinaison biphasique
- d'une première phase incluant le carbonate de calcium, l'acide citrique et l'acide malique dans laquelle l'acide citrique est micronisé et,
- d'une deuxième phase contenant la vitamine D₃ et l'acide citrique pour un montant d'au moins 20% en poids.

2. La composition pharmaceutique selon la revendication 1, dans laquelle la composition comprend de l'acide citrique micronisé dans la première phase ayant une dimension particulaire moyenne comprise entre 150-350 µm, de préférence 250 µm.

3. La composition pharmaceutique selon la revendication 1, dans laquelle la première phase de la composition comprend de l'acide malique qui est utilisé à la place de l'acide citrique pour un montant d'au moins 20% et de préférence 20-60% en poids.

4. La composition pharmaceutique selon la revendication 1, dans laquelle la première phase de la composition comprend de l'acide citrique micronisé dans la plage de 40-80% en poids.

5. La composition pharmaceutique selon la revendication 1, dans laquelle la deuxième phase de la composition comprend de l'acide citrique de préférence dans la plage de 20-60% en poids.

6. La composition pharmaceutique selon la revendication 1, comprenant au moins un excipient pharmaceutiquement acceptable choisi parmi le diluant, le liant, le désintégrant, le lubrifiant, le glissant, l'agent de stabilisation, l'agent aromatisant et l'agent de goût.

7. La composition pharmaceutique selon la revendication 6, dans laquelle l'agent de stabilisation est choisi parmi les antioxydants, les agents chélatants, les agents d'alcalinisation et les photoprotecteurs.

8. La composition pharmaceutique selon la revendication 1, dans laquelle ladite composition comprend:
- du carbonate de calcium dans la plage de 625-12500 mg (correspondant au calcium élémentaire dans la plage de 250-5000 mg)
- de la vitamine D₃ dans la plage de 100-100000 IU,
- de l'acide citrique micronisé pour un montant ne dépassant pas 60% en poids,
- de l'acide malique pour un montant ne dépassant pas 10% en poids,
- du moins un excipient pharmaceutiquement acceptable choisi parmi le diluant, le liant, le désintégrant, le lubrifiant, le glissant, l'agent de stabilisation, l'agent de goût et l'agent aromatisant.

9. La composition pharmaceutique selon la revendication 1, dans laquelle, au moins un désintégrant est l'acide citrique et l'acide malique comme l'acide effervescent et de préférence le carbonate de sodium et/ou le carbonate d'hydrogène de sodium comme la base effervescente.

10. La composition pharmaceutique selon la revendication 9, dans laquelle le désintégrant est de préférence présent pour un montant ne dépassant pas 80% en poids.

11. La composition pharmaceutique selon la revendication 1, dans laquelle ladite composition est sous la forme de dosages telle que le comprimé, la capsule, le comprimé à désintégration rapide, le granule effervescent, le granule à désintégration rapide, le mélange de poudre à désintégration rapide et le mélange de poudre séché pour la préparation du sirop.

12. La composition pharmaceutique selon la revendication 11, dans laquelle ladite composition est sous la forme d'un comprimé effervescent.

13. La composition pharmaceutique selon la revendication 12, dans laquelle la durée de précipitation dans 150 ml d'eau est retardée et l'absorbance de ces comprimés sont au moins 0,4 en spectroscopie UV-Vis à 480 nm dans cet intervalle de temps.

14. La composition pharmaceutique selon la revendication 1, pour l'utilisation afin de prévenir et/ou traiter et/ou supplémenter les maladies métaboliques osseuses.

15. Une méthode pour la préparation d'une composition pharmaceutique selon l'une quelconque des revendications précédentes, dans laquelle,
dans la période de préparation de la première phase :
1) La solution de granulation comprenant de l'eau déionisée et au moins un liant pharmaceutiquement acceptable est préparé;
2) L'acide citrique pour en montant de 40-80% en poids est tamisé de telle façon à ce que sa taille moyenne des particules sera dans la plage de 150-350 µm et de préférence 250 µm;
3) Le carbonate de calcium, l'acide citrique micronisé tamisé, l'acide malique remplacé par le montant de l'acide citrique dans la plage d'au moins 20% et de préférence 20-60% en poids, au moins un liant pharmaceutiquement acceptable et au moins un agent de goût et/ou un agent aromatisant est alimenté dans un granulateur;
4) La composition obtenue est granulée par la solution de granulation;
5) Les granules obtenus sont séchés de telle façon à ce que son humidité sera 0,4% au maximum;
dans la période de préparation de la deuxième phase:
6) La vitamine D₃ est mélangée avec au moins un liant pharmaceutiquement acceptable et/ou un diluant;
7) Le mélange obtenu est combiné avec le reste de l'acide citrique qui est dans la plage de 20-60% en poids, au moins un agent de goût et/ou un agent aromatisant et de préférence au moins un excipient ayant un effet stabilisant;
dans la période de préparation de la composition finale:
8) La composition finale est formée par l'ajout de granules obtenus dans l'étape 5 après tamisage et les ajouter dans le mélange obtenu dans l'étape 7;
9) La composition finale est alimentée dans la presse afin d'être comprimé et les comprimés sont pressés selon les spécifications.
